# EUROPEAN PATENT SPECIFICATION

(11) **EP 3 325 636 B1**
(45) Date of publication and mention of the grant of the patent: **24.04.2024**
(21) Application number: 16828389.3
(22) Date of filing: 18.07.2016
(51) Int. Cl.: C12P 7/06, C12P 7/08, C07H 1/00, C13K 1/02, B09B 3/00, B03B 9/06, A61L 2/07, A61L 11/00, C12P 19/02, C12P 19/14, C13K 13/00, C12P 7/649, B09B 3/80, C12P 7/10

(54) **CONVERSION OF A SOILED POST-CONSUMER CELLULOSIC HYGIENE PRODUCT**
UMWANDLUNG EINER VERSCHMUTZTEN VERBRAUCHTEN HYGIENEARTIKEL
CONVERSION D'UN PRODUIT D'HYGIÈNE POST-CONSOMMATEUR SOUILLÉ

(30) Priority: 17.07.2015 US 201562193701 P
(43) Date of publication of application: 30.05.2018
(73) Proprietor: The Procter & Gamble Company, Cincinnati, OH 45202 (US)
(72) Inventor: LI, Chenlin, Castro Valley, CA 94552 (US); LIANG, Ling, Emeryville, CA 94608 (US); GARDNER, James, Oakland, CA 94609 (US); TANJORE, Deepti, Emeryville, CA 94608 (US)
(74) Representative: P&G Patent Germany
(86) International application number: PCT/US2016/042863
(87) International publication number: WO 2017/015242

(56) References cited:
- US-A- 5 326 477
- US-A- 5 618 003
- US-A1- 2010 112 242
- US-A1- 2012 115 200
- US-A1- 2014 315 258

## Description

### STATEMENT OF GOVERNMENTAL SUPPORT

The invention was made with government support under Contract Nos. DE-AC02-05CH11231 awarded by the U.S. Department of Energy. The government has certain rights in the invention.

### FIELD OF THE INVENTION

The present invention is in the field of deconstruction of post-consumer material.

### BACKGROUND OF THE INVENTION

In the U.S., absorbent hygiene products account for a yearly production of ca.7,500,000 tonnes. Out of this amount, ca. 2,500,000 tonnes/year of high quality cellulose could be reclaimed (sterilized and washed) and upcycled into high- value applications such as biofuels. In Italy, a million ton of municipal solid waste is generated yearly, which contains 30% cellulose materials. As a pioneer of the waste management in Italy, FATER S.p.A. (Pescara, Italy) set up a system for the collection and recycling of used diapers, aiming to demonstrate how the responsible approach to utilize the waste and generate benefits. US 2012/115200 A1 discloses a process for the recovery of ethanol from lignocellulosic biomass. US 2014/315258 A1 discloses processes for converting cellulosic waste, such as municipal solid waste. US 5 326 477 A discloses a process for digesting solid waste such as absorbent pads. US 2010/112242 A1 discloses processes to convert a biomass to produce fuel. US 5 618 003 A discloses processes for the treatment of disposable products. There is a need to better dispose used diapers, and to find a use for them.

### SUMMARY OF THE INVENTION

The invention is set out in the appended set of claims. The present invention provides for a method for deconstructing a post-consumer cellulosic hygiene product composition comprising: (a) providing a soiled post-consumer cellulosic hygiene product composition previously subjected to a high temperature and high pressure process wherein the composition comprises cellulose, cotton, or a mixture thereof, and super absorbent polymers (SAP),
wherein the high temperature and high pressure process is a sterilizing process, wherein the high temperature and high pressure process is an autoclave process, (b) introducing a cellulase and/or a hemicellulase to the soiled post-consumer hygiene product cellulosic composition such that a sugar is produced, (c) separating the sugar from the soiled post-consumer hygiene product cellulosic composition, (d) optionally culturing a microbial strain in a culture using the sugar produced from (b) introducing step as a carbon source such that a biofuel is produced, and (e) optionally separating the biofuel produced in the (d) culturing step from the culture medium.

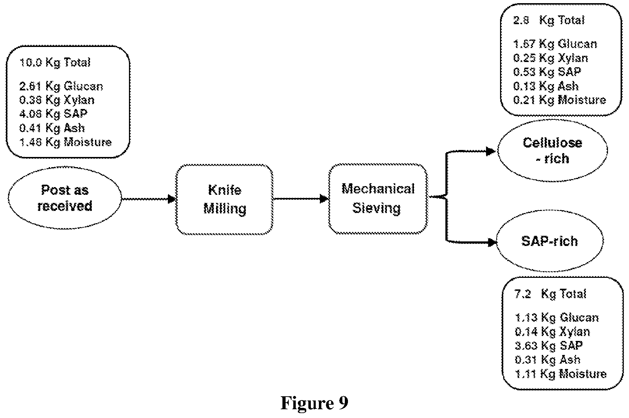

The (a) providing step comprises: (i) milling, such as knife milling the soiled post-consumer cellulosic composition, and (ii) sieving, such as mechanically sieving, the soiled post-consumer cellulosic composition to produce a cellulose-rich fraction and a super absorbent polymer (SAP) fraction. In some embodiments, the cellulose-rich fraction and/or SAP-rich fraction comprise the ratios of the components shown in Figure 9. In some embodiments, the SAP recovery is 50%, or 60%, or 70%, or 80%, or 85%, or 88%, or more from the SAP-rich fraction. In some embodiments, the glucan recovery is 50%, or 60%, or 64%, or more from the cellulose-rich fraction. In some embodiments, the xylan recovery is 50%, or 60%, or 66%, or more from the cellulose-rich fraction.

Also disclosed but not claimed is a composition comprising a biofuel produced using the method of the present invention.

### BRIEF DESCRIPTION OF THE DRAWINGS

The foregoing aspects and others will be readily appreciated by the skilled artisan from the following description of illustrative embodiments when read in conjunction with the accompanying drawings.
Figure 1 shows the experimental schema for Phase 1 as described in Example 1 herein.
Figure 2 shows: (A) the direct enzymatic release of glucose (left) and xylose (right) from "Raw" (virgin cellulose fluff), "Pre" (pre-consumer) and "Post" (post-consumer) materials under 72 h enzymatic hydrolysis at 5% solid loading and 9.2 mg protein/g cellulose, and (B) the effect of solid loading for "Post" material.
Figure 3 shows the preprocessing by milling did not increase enzymatic sugar conversion: (A) glucose conversion of "Raw" material, (C) glucose conversion of "Post" materials, and (D) xylose conversion of "Post" materials.
Figure 4 shows the effect of thermochemical pretreatment on the enzymatic: (A) glucose release, and (B) xylose release from "Raw" material.
Figure 5 shows the effect of thermochemical pretreatment on the enzymatic: (A) glucose release, and (B) xylose release from "Pre" material.
Figure 6 shows the effect of thermochemical pretreatment on the enzymatic: (A) glucose release, and (B) xylose release from "Post" material.
Figure 7 shows the ethanol fermentation of sugar hydrolysates directly released from" (A) 5% "Raw", (B) 5% "Pre", (C) 5% "Post", and (D) 10% "Post".
Figure 8 shows the possible deconstruction routes are better defined. The arrows which can indicated with the " 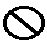 " symbol are deconstruction routes that do not appreciably or significant contribute to deconstruction.
Figure 9 shows one embodiment of the milling and sieving steps.

### DETAILED DESCRIPTION OF THE INVENTION

Before the invention is described in detail, it is to be understood that, unless otherwise indicated, this invention is not limited to particular sequences, expression vectors, enzymes, host microorganisms, or processes, as such may vary. It is also to be understood that the terminology used herein is for purposes of describing particular embodiments only, and is not intended to be limiting.

In order to more fully appreciate the invention the following definitions are provided.

As used in the specification and the appended claims, the singular forms "a," "an," and "the" include plural referents unless the context clearly dictates otherwise. Thus, for example, reference to a "C6 sugar" includes a single C6 sugar as well as a plurality of C6 sugars, either the same (e.g., the same compounds) or different.

In some embodiments, the post-consumer cellulosic composition is diaper. The post-consumer cellulosic composition comprises cellulose, cotton, or a mixture thereof, and super absorbent polymer (SAP), such as polyacrylate. In some embodiments, the amount of polyacrylate can comprises up to about 50%, or more, by weight of the post-consumer cellulosic composition. In some embodiments, the post- consumer cellulosic composition comprises lignin. In other embodiments, the post-consumer cellulosic composition does not comprise lignin. In some embodiments, the diaper is a used diaper. In some embodiments, the used diaper is soiled with an animal waste product, such as urine or feces, or both.

The presence of SAP, such as polyacrylate, (about 50%) can lead to very viscous sugar hydrolysates during enzymatic saccharification, limited mixing, and/or mass transfer at high solid loading, and could potentially inhibit the activity of any enzyme and microorganisms for downstream bioprocessing. Cellulose and SAP fractions obtained by milling and/or separation have different material morphology, toughness, particle size and density. The cellulose fractions are fibrous and low in density, while the SAP fractions have a more uniform particle size and higher density.

Knife milling and mechanical sieving of the post-consumer cellulosic composition comprising SAP, such as polyacrylate, improves the removing of the SAP, such as polyacrylate, which in turn improves the solid loading of post-consumer materials for sugar conversion.

The present invention also provides for a method comprising milling and mechanical separating, based on particle size and/or density, resulting in the removing or separating of up to about 88% of the SAP, such as polyacrylate, from the post-consumer cellulosic composition. The reduction of the SAP, such as polyacrylate, content from the post-consumer cellulosic composition increases the mixing and mass transfer during the enzymatic saccharification step at high solid loadings and facilitates the conversion to high titer fermentable sugars, biofuels and bioproducts.

The soiled post-consumer cellulosic composition is sterilized, by a high temperature and high pressure process. The providing step (a) comprises sterilizing the soiled post-consumer cellulosic composition using the high temperature and high pressure process, and optionally removing any nonbiodegradable material in the soiled post-consumer cellulosic composition. In some embodiments, the non-biodegradable material is polyacrylate or plastic. The high temperature and high pressure process is an autoclave process. In some embodiments, the post-consumer cellulosic composition, prior to being subjected to a high temperature and high pressure process, is biohazardous.

In some embodiments, the post-consumer cellulosic composition has not been subjected to an acid pretreatment.

The cellulase and hemicellulase used in the present invention can be any enzyme that has a cellulase activity or a hemicellulase activity, respectively. The cellulase and hemicellulase can be part of an enzyme cocktail comprising one or more cellulases, one or more hemicellulases, or a mixture thereof. The cellulase, or hemicellulose, can be a thermostable cellulase or a non-thermostable cellulase, thermostable hemicellulase or a non-thermostable hemicellulase, such as those taught in U.S. Patent Application Pub. No. 2012/0129227 The cellulase can be an endocellulase exocellulase (or cellobiohydrolase), cellobiase, oxidative cellulase, cellulose phosphorylase, or a mixture thereof. The cellulase can be an endo-I,4-beta-D-glucanase (beta-l,4-glucanase, beta-I,4-endoglucan hydrolase, endoglucanase D, l,4-(l,3,l,4)-beta-D-glucan 4-glucanohydrolase), carboxymethyl cellulase (CMCase), avicelase, or the like. The cellulase and/or hemicellulase can be part of a cellulase cocktail, such as CTec2 and HTec2, which are commercially available from Novozymes (Bagsvaerd, Denmark). A cellulase is an enzyme, or a homologous enzyme thereof, that has an enzymatic activity for hydrolyzing cellulose, hemicelluloses, or lignocelluloses. A cellulase includes, but is not limited to, an endoglucanase, exoglucanase, or P-I,4-D-glucosidase, or a homologous enzyme thereof. A hemicellulase can be an endoxylanase. A thermostable cellulase is a cellulase that has an optimal temperature that is equal to or more than 65 °C.

Thermostable cellulases or hemicellulases suitable for use in the present invention, include are any thermostable cellulase or hemicellulase from the genus Anaerocellu, Bacillus, *Rhodothermus, Thermotoga, Sulfolobus*, or *Pyrococcus.* Suitable species of the genus *Anaerocellu* include A. *thermophilum.* Suitable species of the genus *Bacillus* include *B. subtilus.* Suitable species of the genus *Rhodothermus* include *R. marinus.* Suitable species of the genus *Thermotoga* include *T. maritima, T. neapoltana,* and *T. subterranea.* Suitable species of the genus *Sulfolobus* include *S*. *solfataricus* MT4, *S. acidocaldarius*, and *S*. *shibatae.* Suitable species of the genus *Pyrococcus* include *P. horikoshii, P. horicoshi, P. woesei,* and *P. furiosus.* In some embodiments, the thermostable cellulase, or thermostable hemicellulase, is a cellulase, or hemicellulase, obtained from or native to a hyperthermophlic microorganism, an extremophilic microorganism, or thermophilic microorganism. In some embodiments, the thermostable cellulase is a thermophilic cellulase. In some embodiments, the thermostable hemicellulase is a thermophilic hemicellulase. In some embodiments, the thermostable cellulase is a thermostable endoglucanase or a thermophilic endoglucanase. Some of the suitable thermostable cellulases are listed in Table 1.

**Table 1. Source microorganisms and properties of thermostable cellulases.**

| Organism | Enzyme properties | | References |
|---|---|---|---|
| | Optimal temperature (°C) | Optimal pH | |
| *Anaerocella* | S5-90 | 5.0-6.6 | Zverlov et al. (1998) |
| *Bacillus subtilis* | 65-70 | 5.0-6.5 | Mawadza et al. (2000) |
| *Pyrococcus furiosus* | 102-105 | - | Kengen et al. (1993) |
| *Pyroccus* | 97 | - | Ando et al. (2002) |
| *Rhodothermus marinus* | 95 | 6.5-8.0 | Hreggvidsson et. al. (1996) |
| *Thermofoga* MSBS | 95 | 6.0-7.0 | Bronnenmeier et al. (1995) |
| *Thermofoga neapoliana* (EndocellulaseA) | 95 | 6.0 | Bok et al. (1996) |
| *Thermofoga neapoliana* (EndocellulaseB) | 106 | 6.0-6.6 | Bok et al. (1998) |

Zverlov, V., Riedel, K. and Bronnenmeier, K., 1998. Properties and gene structure of a bifunctional cellulytic enzyme (CelA) from the extreme thermophile Anaerocellum thermophilum with separate glycosyl hydrolase family 9 and 48 catalytic domains. Microbiology 144, pp. 457-465.

Mawadza, C., Hatti-Kaul, R., Zvauya, R. and Mattiasson, B., 2000. Purification and characterization of cellulases produced by two Bacillus strains. J. Biotechnol. 83, pp. 177-187.

Kengen, S., Luesink, E., Stams, A. and Zehnder, A., 1993. Purification and characterization of an extremely thermostable β-glucosidase from the hyperthermophilic archaeon Pyrococccus furiosus. Eur. J. Biochem. 213, pp. 305-312.

Ando, S., Ishida, H., Kosugi, Y. and Ishikawa, K., 2002. Hyperthermostable endoglucanase from Pyrococcus horikoshi. Appl. Environ. Microbiol. 68, pp. 430-433.

Hreggvidsson, G.O., Kaiste, E., Holst, O., Eggertsson, G., Palsdottir, A. and Kristjansson, J.K., 1996. An extremely thermostable cellulase from the thermophilic eubacterium Rhodothermus marinus. Appl. Environ. Microbiol. 62, pp. 3047-3049.

Bronnenmeier, K., Kern, A., Libel, W. and Staudenbauer, W., 1995. Purification of Thermotoga maritema enzymes for the degradation of cellulose materials. Appl. Environ. Microbiol. 61, pp. 1399-1407.

Bok, J., Goers, S. and Eveleigh, D., 1994. Cellulase and xylanase systems of Thermotoga neapolitana. ACS Symp. Ser. 566, pp. 54-65.

Bok, J., Dienesh, A., Yernool, D. and Eveleigh, D., 1998. Purification, characterization and molecular analysis of thermostable cellulases CelA and CelB from Thermotoga neapolitana. Appl. Environ. Microbiol. 64, pp. 4774-4781.

In some embodiments, the sugar is a monomeric sugar, such as a C6 sugar or a C5 sugar. In some embodiments, the C6 sugar is glucose. In some embodiments, the C5 sugar is xylose.

The microbial strain can be a prokaryote or a eukaryote. The prokaryote can be an eubacteria or archaebacterial. In some embodiments, the eukaryote is a fungal strain or an algae. In some embodiments, the microbial strain is a recombinantly engineered strain that has been engineered to produce a biofuel.

In some embodiments, the biofuel is any organic compound capable of combustion, such as ethanol, levunic acid, algae, or bisabolene.

In some embodiments, the microbial strain is any suitable microorganism or cell, and the biofuel is any combustible organic compound(s) synthesized or produced by the microorganism or cell thereof, disclosed in U.S. Patent Nos. 7,670,825; 7,736,882; 7,915,026; 7,985,567; 8,097,438; 8,114,645; 8,163,980; 8,257,957; 8,288,147; 8,420,833; 8,535,916; 8,569,023; 8,759,632; 8,765,403; 8,828,684; 8,852,902; 9,040,282; and U.S. Patent Application Pub. Nos. 2015/0087042, 2015/0044747, 2015/0044734, 2014/0370595, 2014/0295517, 2014/0134689, 2014/0038248, 2014/0030789, 2013/0280766, 2013/0267696, 2013/0267012, 2013/0245339, 2013/0115668, 2013/0059295, 2013/0052692, 2012/0288905, 2012/0219998, 2012/0219971, 2012/0190090, 2012/0142979, 2012/0115195, 2011/0229958, 2011/0097769, 2011/0021790, 2011/0014667, 2011/0008829, 2010/0242345, 2010/0218283, 2010/0205855, 2010/0180491, and 2010/0170148 (hereby incorporated by reference in regards in the microorganism or cell, and the combustible organic compound(s) synthesized or produced by the microorganism or cell thereof).

It is to be understood that, while the invention has been described in conjunction with the preferred specific embodiments thereof, the foregoing description is intended to illustrate and not limit the scope of the invention. Other aspects, advantages, and modifications within the scope of the invention will be apparent to those skilled in the art to which the invention pertains.

The invention having been described, the following examples are offered to illustrate the subject invention by way of illustration, not by way of limitation.

### EXAMPLE 1

The aim of the following experiments is to evaluate the feasibility of converting post-consumer hygiene materials and the effects of milling, pretreatment, saccharification, and fermentation on these materials. The experiments are conducted using bench top reactors. The project is started with knife milling or ball milling to reduce the particle size and enhance the surface area, followed by the thermochemical pretreatment in the 25 mL tube reactors. The pretreated materials are saccharified in flasks using the Novozymes (Bagsvaerd, Denmark) cellulase cocktails CTec2 and HTec2 into monomeric sugars. Based on the sugar conversion and inhibitor release from the pretreatment, the hydrolysates produced under selected condition are fermented into ethanol by yeast in the flasks.

FATER S.p.A. (Pescara, Italy) has developed an innovative autoclave process for Absorbent Hygiene Product (AHP) waste treatment. Such process will sanitize, de-compose, and dry the post-consumer AHP, leading to the recovery of its valuable components (mostly high-quality cellulose and specialty plastics). The innovation resides into a specifically designed steam process, which combines for the first time steam sterilizing with efficient drying within a single autoclave. As a result it can manage the high AHP waste humidity levels and dries the output stream to about 20% or below, as compared to contemporary autoclaves that result in product humidity of well over 50%. The technical nutrients stream (Plastics) will be upcycled into valuable products. The dried biological nutrients stream (cellulose/organic materials) have some identified outlets as feedstock for the packaging/paper industry and, being organic material that has a high caloric value, as input for pyro-gasification (IGCC) process to generate renewable electricity and steam. However other outlets/technologies to gain higher value from the recovered raw materials are being pursued.

The substrate of interest in this project lacks lignin due to the sulphite/sulphate process performed prior to the product manufacture. Also, after product usage, the substrate undergoes the autoclave sterilization, which itself acts as a mild thermochemical pretreatment. However, the substrate remains fibrous and may require a mechanical or thermochemical pretreatment to disrupt the fibrous structure followed by enzymatic saccharification to release monomeric glucose, which can be converted to ethanol through yeast fermentation. In addition, prior to the conversion process, the particle size reduction is also required to accommodate the limitations associated with mixing in pretreatment, saccharification and fermentation reactors. Besides the post-consumer materials, the virgin materials as well as pre-consumer materials are also taken into consideration for their characteristics of conversion to ethanol as the controls to allow better understanding of economic analysis of product manufacture and sterilization processes.

### 1 Purpose

The rationale for the project has been to (1) understand the viability of rendering fermentable sugar from the cellulosic fraction of sterilized, post-consumer hygiene products, and (2) the further validation of sugar fermentability for bioethanol production. Typically, these waste products would simply find their way to a landfill. An application of these sterilized, post-consumer hygiene products is as a promising renewable cellulosic feedstock for biofuels and biochemical production through microbial fermentation.

### 2 Executive Summary

The results to date of the Phase 1-3 testing are summarized as follows:

### 2.1 Phase 1: Determining the Deconstruction Routes

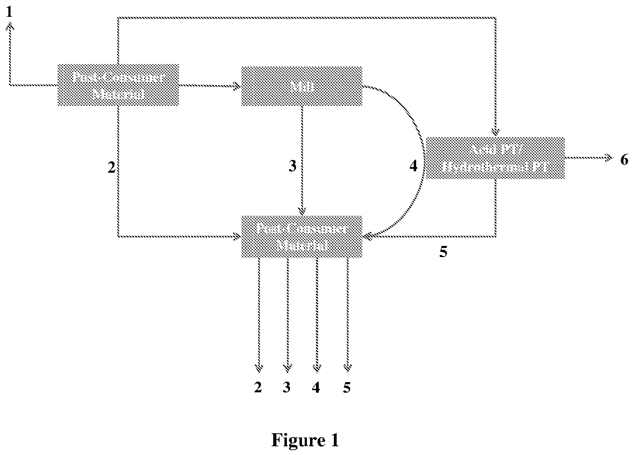

**Goal:** determine the minimum number of unit operations needed to yield monomeric sugar from mock, post-consumer, sterilized cellulosic material [Post]. See Figure 1.

**Result:** From the data, it is already clear that milling the material is an unnecessary step. Thermochemical pretreatment, i.e., dilute acid and hydrothermal, didn't show improvement on the enzymatic saccharification either. The as received Post converted readily to monomeric glucose with moderate cellulase enzyme loading, even without particle size reduction and aqueous pretreatment. The material was quite viscous, which may point to the importance of stirred tank design with effective impeller configuration in the consideration of mass transfer at larger scales. The solid loading was increased by 2 times to obtain higher sugar concentration, although not required in the proposal scope, and the results show a linear increase in monomeric sugar production. These outcome show great promises for developing an economically viable process. The minimum unit operation for deconstruction will be direct enzymatic saccharification.

### 2.2 Phase 2: Upstream effects

**Goal:** determine the effects of upstream processes and additives by subjecting virgin cellulose fluff [**Raw**] and the pre-consumer material [**Pre**] to the subset of unit operations from 2.1.

**Result:** In practice, it proved more sensible to perform most of these experiments in parallel with those from 2.1. The primary observation was that the polyacrylate water absorbent had a strong impact on the viscosity of the material, which in turn complicated the ability to perform effective enzymatic saccharification. This will likely impact the economics of the emergent process. Addition of polyacrylate showed a negative effect on the saccharification in both Pre and Post materials, which requires mechanical separation process prior the saccharification.

### 2.3 Phase 3: Refine the process

**Goal:** Compare the acid and hydrothermal pretreatment on the enzymatic saccharification, and test the fermentability of sugars produced from the refined deconstruction process for ethanol production.

**Result:** The data show that acid and hydrothermal pretreatment did not efficiently stimulate glucose yields for Raw and Pre materials. A decrease of glucose and xylose yields were observed for pretreated Post materials. For all three materials, direct enzymatic saccharification had either faster kinetics, or higher sugar conversion. Sugars from all three materials are fermentable by yeast with the conversion yields between 0.30-0.37 g ethanol/g glucose.

### 3 Data: Unit operations, upstream effect and fermentation.

### 3.1 Determining the Deconstruction Routes

In order to account for the influence of various unit operations the first round of experimentation will consist of testing for sugar yield in the following 6 conditions on mock **Post**-consumer material as well as **Raw** and **Pre**-consumer:
1. Untreated
2. Enzyme only
3. Milling + Enzyme
4. Milling + Acid pretreatment + Enzyme
5. Acid + Enzyme
6. Acid only

Figure 1 shows the experimental schema for Phase 1.

### 3.2 Post-consumer material has roughly 30% polysaccharide by weight

The feedstocks used in the experiments are shown in Table 2. "Raw" indicates pure cellulose. "Pre" indicates pre-use cellulose plus absorbent. "Post" indicates post-use cellulose plus absorbent. The municipal solid waste (MSW) materials show different characteristics in comparison with lignocellulosic biomass: low density and poor distribution. The results indicate that the "as received" materials have the highest mass closure (which is due to the presence of the polyacrylate). The "Pre" and "Post" materials should be handled with caution to maintain uniformity.

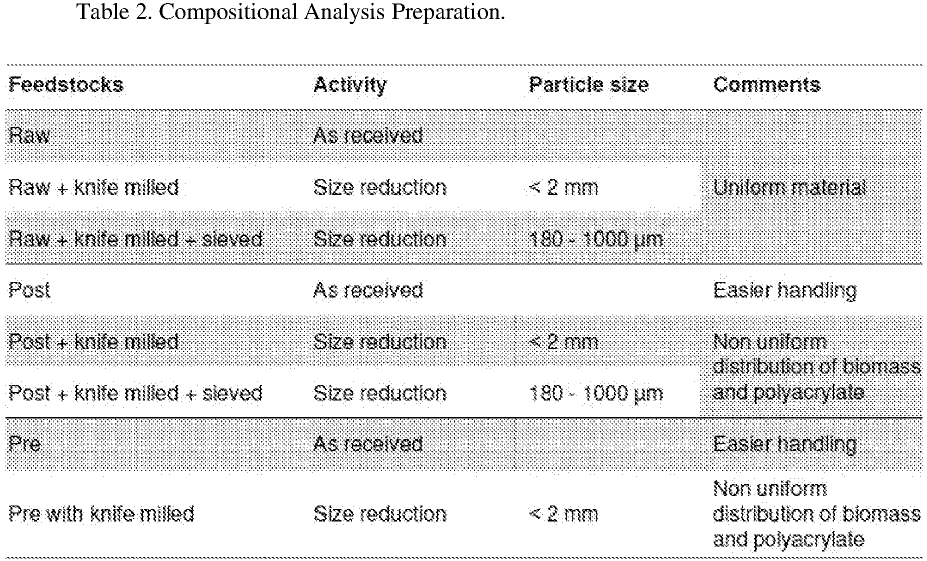

Owing to the presence of polyacrylate, other components, and perhaps a partial decomposition during sterilization, the weight of the "Post" feedstock is roughly 70% other components. This finding contrasted with both "Raw" and "Pre" materials, which contained relatively higher levels of polysaccharide (see Table 3). The "Pre" and "Post" materials have lower polysaccharide content due to the presence of polyacrylate. The "Post" material has the lowest polysaccharide content. This may be caused by partial decomposition during sterilization.

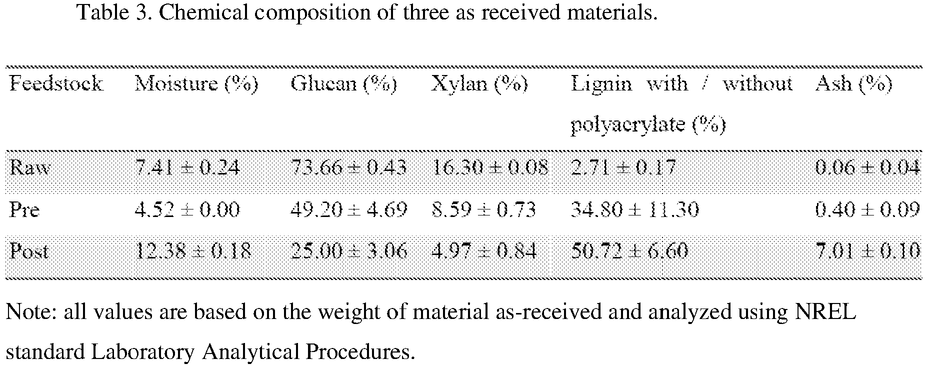

3.3 Direct enzymatic saccharification experiments show high sugar conversion for all three materials

Addition of polyacrylate poses upstream effect on the materials as given by the decreased sugar conversion of "Pre". Sterilization may likely facilitate the saccharification as shown by the increased sugar conversion of "Post" in comparison with "Pre" material, which may attribute to the reduction in the degree of polymerization in cellulose structure during sterilization process.

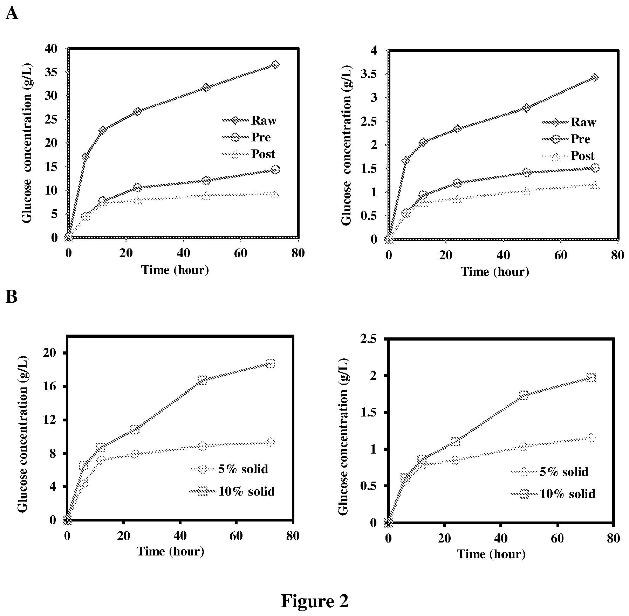

Figure 2, Panel A shows the direct enzymatic release of glucose (left) and xylose (right) from Raw, Pre and Post materials under 72 h enzymatic hydrolysis at 5% solid loading and 9.2 mg protein/g cellulose. The results show that the three materials shown in Figure 2 can be successfully converted to monomeric sugar through direct enzymatic saccharification. The upstream effect accounts for the lower sugar concentrations obtained for the "Pre" and "Post" materials. The reaction condition is shown in Table 4. The various sugar yields are shown in Table 5. Figure 2, Panel B shows the effect of solid loading for "Post" material. The monomeric sugar concentration and solid loading indicate a similar variation trend. The results show that with solid loading doubling, the monomeric sugar concentration nearly doubled as well.

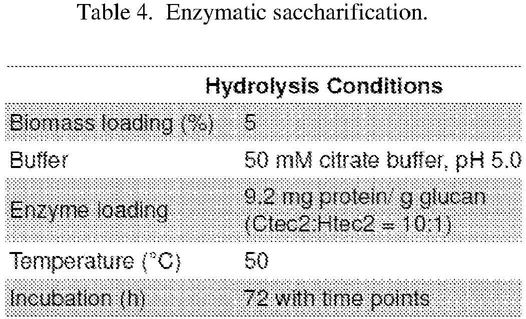

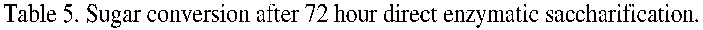

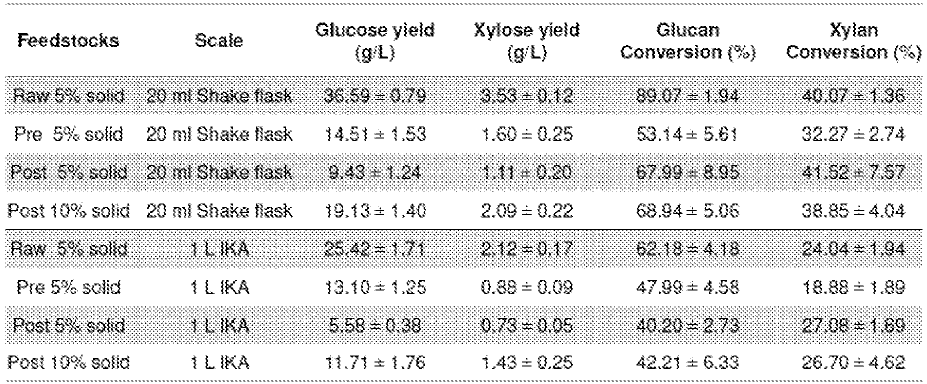

Both the 20 ml scale in shake flasks and the 1 L scale in bioreactors show good sugar conversion. The upstream effect accounts for the lower glucan and xylan conversion for the "Post" material and lowest conversion in the "Pre" material.

### 3.4 Milling is unnecessary for high enzymatic conversion to glucose from both Raw and Post materials

The feedstock can be milled, such as knife milling and ball milling" prior to saccharification. Knife milling can involve a 2 mm screen for up to 5 minutes. Ball milling can involve the use of a ball mill analytical tool using 650 rpm for 20 minutes. However, while "Raw" feedstock density dramatically increases with milling, "Post" feedstock density changes very little after milling.

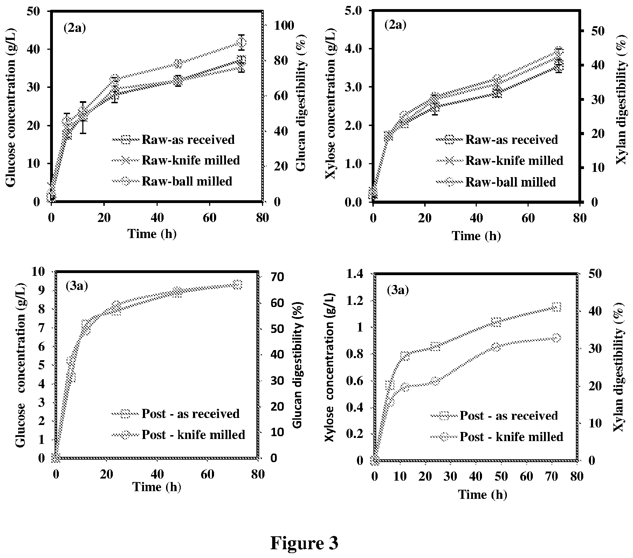

Figure 3 shows the preprocessing by milling did not increase enzymatic sugar conversion: (A) glucose conversion of "Raw" material, (B) xylose conversion of "Raw" material, (C) glucose conversion of "Post" materials, and (D) xylose conversion of "Post" materials.

### 3.5 Acid and hydrothermal pretreatment experiments did not increase the enzymatic conversion.

Dilute acid pretreatment comprises a pretreatment using 1 % sulfuric acid at 120 °C for 15 minutes at 10% solid loading. Hydrothermal pretreatment comprises a pretreatment at 120 °C for 15 minutes at 10% solid loading.

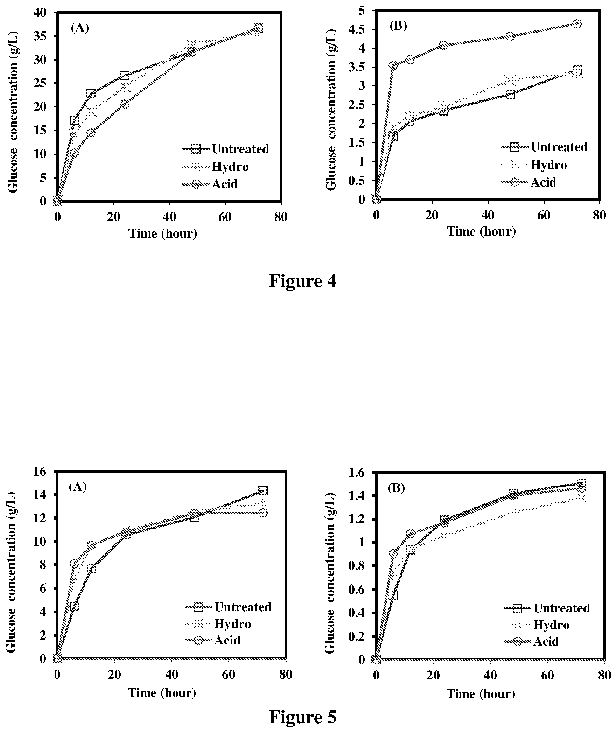

Figure 4 shows the effect of thermochemical pretreatment on the enzymatic: (A) glucose release, and (B) xylose release from "Raw" material. In the shake flask scale, pretreatment did not efficiently stimulate glucose yield. The dilute acid pretreatment resulted in an increase in xylose yield for the "Raw" material.

Figure 5 shows the effect of thermochemical pretreatment on the enzymatic: (A) glucose release, and (B) xylose release from "Pre" material. The results show there is little increase in sugar yield, and that both conversion kinetic and sugar yield had similar trends.

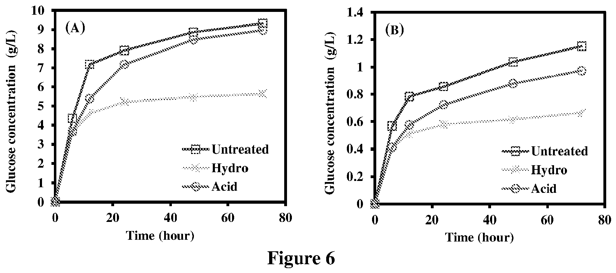

Figure 6 shows the effect of thermochemical pretreatment on the enzymatic: (A) glucose release, and (B) xylose release from "Post" material. The results show a decrease of glucose and xylose yields for pretreated materials. Direct enzymatic saccharification showed faster kinetics, or higher sugar conversion.

Results show that Enzymatic only is the desired deconstruction route for all three materials. Table 6 shows the thermochemical pretreatment and sugar conversion. The results indicate that the value of thermochemical pretreatment is limited, and direct enzymatic saccharification is recommended.

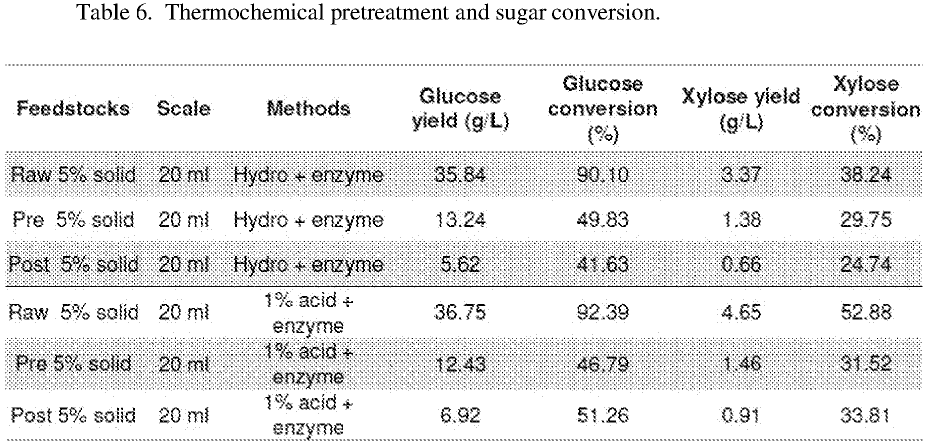

### 3.6 Bioethanol production by batch yeast fermentation

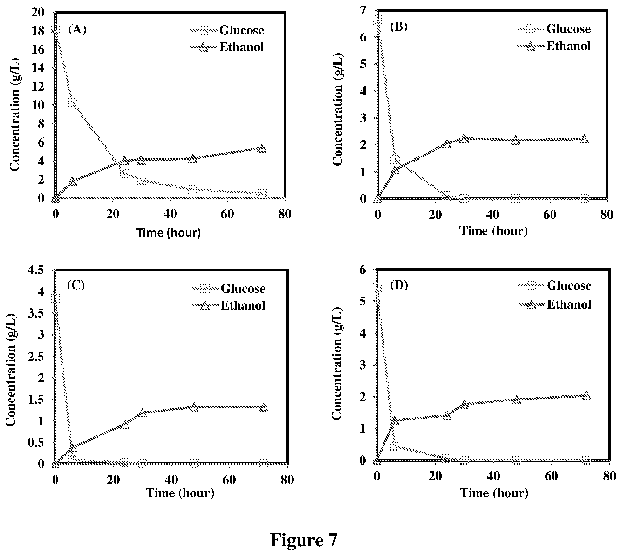

Figure 7 shows the ethanol fermentation of sugar hydrolysates directly released directly from" (A) 5% "Raw", (B) 5% "Pre", (C) 5% "Post", and (D) 10% "Post". The batch fermentation in shake flasks comprises using *Saccharomyces cerevisiae* ATCC 20389. The seed medium comprises YPD (Yeast Extract-Peptone-Dextrose) medium (20 g/L glucose, 10 g/L yeast extract, and 20 g/L peptone). The fermentation medium comprises 100 mL of modified YPD medium (enzymatic hydrolysate, 10 g/L yeast extract, and 20 g/L peptone) in each shake flask. The incubation was at temperature 30 °C, with 250 rpm agitation, for 72 hours.

Fermentation results show that all the sugar hydrolysates released from direct enzymatic hydrolysis of three materials can be fermented to produce ethanol with 0.30 - 0.37 g ethanol /g glucose yields. The reaction is as follows:

(C₆H₁₀O₅)*n* (Cellulose, 162 g/mol) + *n* H₂O → *n* C₆H₁₂O₆ (Glucose 180 g/mol) → 2*n* C₂H₅OH (Ethanol 46 g/mol) + 2*n* CO₂

### 4 Conclusions

AHP can be hydrolyzed to sugars, and then fermented to bioethanol or any other biofuel. The results suggest an economically feasible process with the following observations: (1) milling is not essential, (2) moderate cellulase concentrations can efficiently hydrolyze Post feedstock, (3) thermochemical pretreatment is not necessary. The enzymatic only approach has been shown to be the desired deconstruction route for all three materials. The promising information given by the batch yeast fermentation is the sugars produced from direct enzymatic hydrolysis can be fermented to bioethanol with decent yields.

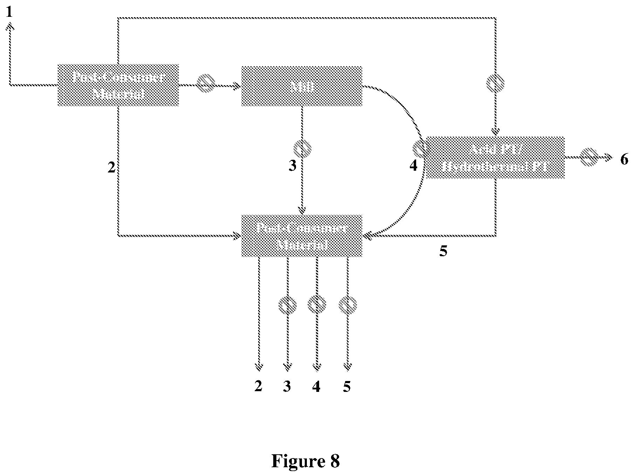

In the light of these observations, the following optimization approach is proposed for an efficient MSW to bioethanol conversion: (1) remove polyacrylate mechanically prior to the saccharification, (2) review the sterilization process (temperature, time, wetness, pressure) to tailor the existing process for efficient conversion, (3) explore higher solid loading for saccharification, (4) further optimize the fermentation conditions, and (5) perform the scale up validation. Figure 8 shows the process steps have been identified as not appreciably or significantly contributing to the deconstruction of the post-consumer material.

### EXAMPLE 2

The aim of the following experiments is to evaluate the feasibility of converting post-consumer hygiene materials by separation, saccharification, and fermentation. The experiments are conducted using bench top reactors as well as scale-up bioreactors and fermenters. The project scope includes: 1) techno-economic analysis by using data generated, and the process optimization data, 2) process optimization to increase the solid loading to obtain high titer fermentable sugars, and 3) evaluation of the actual post-consumer material with the optimized conversion process to bioethanol.

### Process Optimization

There are three objectives: (1) to test the feasibility of mechanical removal of the polyacrylate and potentially improve the downstream process, (2) to improve enzymatic saccharification and fermentation process to obtain high sugar and ethanol titers, and (3) to investigate the scale-up effects.

For post-consumer materials, the presence of polyacrylate leads to very viscous sugar hydrolysates during enzymatic saccharification, limited mixing and mass transfer at high solid loading, and likely poses inhibition effects on the enzyme and microorganisms. It is proposed to test the feasibility of the mechanical screening of polyacrylate, and explore and optimize integration with milling and separation equipment. Cellulose and polyacrylate fractions show different material morphology, toughness, particle size and density. The cellulose materials are fibrous and low in density, while polyacrylate has a more uniform particle size and higher density. It is proposed to apply 1 run of mechanical sieving to investigate the feasibility of polyacrylate particle removal. This should help improve the solid loading of post-consumer materials for sugar conversion.

The saccharification process is also optimized to obtain high solid loading, and further optimize the fermentation process for bioethanol production. These tests are first at the flask scale to determine the optimal condition, and then at the reactor level (2L) to evaluate the scale up effects. See Table 7.

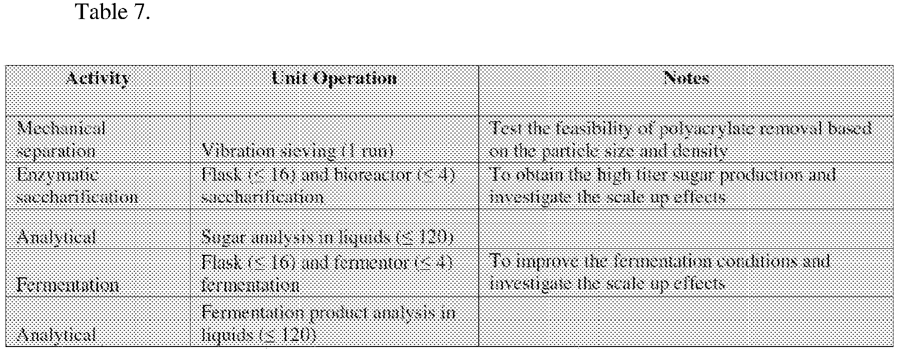

The post-consumer material is sterilized to render the material biologically inactive. Compositional analysis, enzymatic saccharification and yeast fermentation for ethanol production, and comparison of the results with mock post-consumer materials are performed. The potential inhibitory effects resulting from existing consumer hygiene product handling processes on sugar conversion and ethanol production is investigated. The data generated serves as inputs for the analysis to reflect the effects of the actual materials. See Table 8.

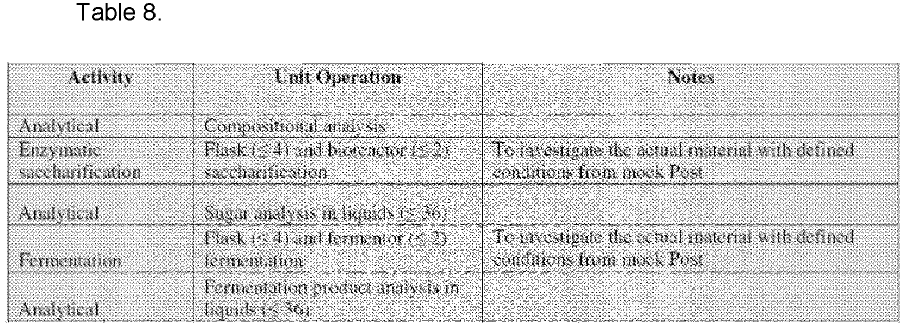

The material used contains polyacrylates. The effect of polyacrylate acid sodium salt, or its inhibition on enzymatic saccharification and microbial fermentation is determined. The facility equipment use comprises: (1) saccharification reactor: 50 mL flasks and 2L IKA reactors, incubator with controlled temperature, sampling points at 0, 6, 24, and 72 hours; (2) fermentation reactors: 50 mL flasks and 2L fermentors incubator with controlled temperature, sampling points at 0, 6, 24, and 72 hours; (3) HPLC to measure glucose yield, furfural release, and ethanol production; and (4) mechanical sieve.

This project validates the concept of the solid waste to biofuel conversion and provides an analysis for using post-consumer AHP material as a renewable source for biofuels and renewable chemicals.

## Claims

1. A method for deconstructing a post-consumer cellulosic hygiene product composition comprising:
(a) providing a soiled post-consumer cellulosic hygiene product composition previously subjected to a high temperature and high pressure process,
wherein the composition comprises cellulose, cotton, or a mixture thereof, and super absorbent polymers (SAP),
wherein the high temperature and high pressure process is a sterilizing process, wherein the high temperature and high pressure process is an autoclave process,
(b) introducing a cellulase and/or a hemicellulase to the soiled post-consumer cellulosic hygiene product composition such that a sugar is produced,
(c) separating the sugar from the soiled post-consumer hygiene product cellulosic composition,
(d) optionally culturing a microbial strain in a culture medium using the sugar produced from (b) introducing step as a carbon source such that a biofuel is produced, and
(e) optionally separating the biofuel produced in the (d) culturing step from the culture medium,
wherein the providing step a) further comprises the steps of: i) milling the soiled post-consumer cellulosic hygiene product composition and ii) sieving, such as mechanical sieving, the soiled post-consumer cellulosic hygiene product composition to produce a cellulose-rich fraction and a super absorbent polymers (SAP) fraction.

2. The method of claim 1, wherein the post-consumer cellulosic composition is diaper.

3. The method of claim 1, wherein the cellulase and/or the hemicellulase are part of an enzyme cocktail comprising one or more cellulases, one or more hemicellulases, or a mixture thereof

4. The method of claim 1, wherein the post-consumer cellulosic composition has not been subjected to an acid pretreatment.

5. The method of claim 1, wherein the sugar is a C6 sugar or a C5 sugar, or both.

6. The method of claim 5, wherein the C6 sugar is glucose.

7. The method of claim 5, wherein the C5 sugar is xylose.

8. The method of claim 1, wherein the biofuel is ethanol.

## Patentansprüche

1. Verfahren zum Dekonstruieren einer gebrauchten cellulosischen Hygieneproduktzusammensetzung, umfassend:
(a) Bereitstellen einer verschmutzten gebrauchten cellulosischen Hygieneproduktzusammensetzung, die zuvor einem Hochtemperatur- und Hochdruckprozess ausgesetzt wird,
wobei die Zusammensetzung Cellulose, Baumwolle oder eine Mischung davon und Superabsorptionspolymere (SAP) umfasst,
wobei der Hochtemperatur- und Hochdruckprozess ein Sterilisationsprozess ist, wobei der Hochtemperatur- und Hochdruckprozess ein Autoklavenprozess ist,
(b) Einbringen einer Cellulase und/oder einer Hemicellulase auf die verschmutzte gebrauchte cellulosische Hygieneproduktzusammensetzung derart, dass ein Zucker erzeugt wird,
(c) Trennen des Zuckers von der verschmutzten gebrauchten cellulosischen Hygieneproduktzusammensetzung,
(d) wahlweise Züchten eines mikrobiellen Stammes in einem Kulturmedium unter Verwendung des Zuckers, der aus dem (b) Einbringungsschritt erzeugt wird, als eine Kohlenstoffquelle, derart, dass ein Biokraftstoff erzeugt wird, und
(e) wahlweise Trennen des Biokraftstoffs, der in dem (d) Züchtungsschritt erzeugt wird, aus dem Kulturmedium,
wobei der Bereitstellungsschritt a) ferner die Schritte umfasst: i) Mahlen der verschmutzten gebrauchten cellulosischen Hygieneproduktzusammensetzung und ii) Sieben, wie mechanisches Sieben, der verschmutzten gebrauchten cellulosischen Hygieneproduktzusammensetzung, um eine cellulosereiche Fraktion und eine Superabsorptionspolymerfraktion (SAP-Fraktion) zu erzeugen.

2. Verfahren nach Anspruch 1, wobei die gebrauchte cellulosische Zusammensetzung eine Windel ist.

3. Verfahren nach Anspruch 1, wobei die Cellulase und/oder die Hemicellulase Teil eines Enzymcocktails sind, umfassend eine oder mehrere Cellulasen, eine oder mehrere Hemicellulasen oder eine Mischung davon.

4. Verfahren nach Anspruch 1, wobei die gebrauchte cellulosische Zusammensetzung nicht einer sauren Vorbehandlung ausgesetzt wurde.

5. Verfahren nach Anspruch 1, wobei der Zucker ein C6-Zucker oder ein C5-Zucker oder beides ist.

6. Verfahren nach Anspruch 5, wobei der C6-Zucker Glucose ist.

7. Verfahren nach Anspruch 5, wobei der C5-Zucker Xylose ist.

8. Verfahren nach Anspruch 1, wobei der Biokraftstoff Ethanol ist.

## Revendications

1. Procédé de déconstruction d'une composition de produit d'hygiène cellulosique post-consommation comprenant :
(a) la fourniture d'une composition de produit d'hygiène cellulosique post-consommation souillé précédemment soumise à un processus à haute température et haute pression,
dans lequel la composition comprend de la cellulose, du coton, ou un mélange de ceux-ci, et des polymères superabsorbants (SAP),
dans lequel le processus à haute température et haute pression est un processus de stérilisation, dans lequel le processus à haute température et haute pression est un processus autoclave,
(b) l'introduction d'une cellulase et/ou d'une hémicellulase dans la composition de produit d'hygiène cellulosique post-consommation souillé de telle sorte qu'un sucre est produit,
(c) la séparation du sucre de la composition cellulosique de produit d'hygiène de post-consommation souillé,
(d) la culture facultative d'une souche microbienne dans un milieu de culture à l'aide du sucre produit à partir de l'étape d'introduction (b) en tant que source de carbone de telle sorte qu'un biocarburant est produit, et
(e) la séparation facultative du biocarburant produit dans l'étape de culture (d) du milieu de culture,
dans lequel l'étape de fourniture a) comprend en outre les étapes consistant à : i) broyer la composition de produit d'hygiène cellulosique post-consommation souillé et ii) tamiser, par exemple tamiser mécaniquement, la composition de produit d'hygiène cellulosique post-consommation souillé afin de produire une fraction riche en cellulose et une fraction de polymères superabsorbants (SAP).

2. Procédé selon la revendication 1, dans lequel la composition cellulosique post-consommation est une couche.

3. Procédé selon la revendication 1, dans lequel la cellulase et/ou l'hémicellulase font partie d'un cocktail enzymatique comprenant une ou plusieurs cellulases, une ou plusieurs hémicellulases, ou un mélange de celles-ci.

4. Procédé selon la revendication 1, dans lequel la composition cellulosique post-consommation n'a pas été soumise à un prétraitement acide.

5. Procédé selon la revendication 1, dans lequel le sucre est un sucre en C6 ou un sucre en C5, ou les deux.

6. Procédé selon la revendication 5, dans lequel le sucre en C6 est le glucose.

7. Procédé selon la revendication 5, dans lequel le sucre en C5 est le xylose.

8. Procédé selon la revendication 1, dans lequel le biocarburant est l'éthanol.
